# EUROPÄISCHE PATENTSCHRIFT

(11) **EP 4 338 661 B1**
(45) Veröffentlichungstag und Bekanntmachung des Hinweises auf die Patenterteilung: **23.04.2025**
(21) Anmeldenummer: 22196279.8
(22) Anmeldetag: 19.09.2022
(51) Int. Cl.: A61B 5/00, A61C 9/00

(54) **VAKUUMFOLIE ZUM SCANNEN EINER MUNDHÖHLE**
VACUUM FILM FOR SCANNING AN ORAL CAVITY
FEUILLE SOUS VIDE POUR BALAYER UNE CAVITÉ BUCCALE

(43) Veröffentlichungstag der Anmeldung: 20.03.2024
(73) Patentinhaber: Ivoclar Vivadent AG, 9494 Schaan (LI)
(72) Erfinder: Lichtensteiger, Markus, 9462 Montlingen (CH)
(74) Vertreter: Baldus, Oliver

(56) Entgegenhaltungen:
- EP-A1- 3 130 885
- US-A1- 2005 226 912
- US-A1- 2017 007 386

## Beschreibung

Die vorliegende Erfindung betrifft eine Vakuumfolie zum Scannen einer Mundhöhle und Verfahren zum Scannen einer Mundhöhle.

Die Form von Mundhöhlen, die keine oder nur wenige Zähne aufweisen, kann nur schlecht mittels Intraoralscannern erfasst werden, da die Mundschleimhaut nur geringe Konturen aufweist.

Dokument EP 3 130 885 A1 offenbart eine Vakuumfolie zum Scannen einer Mundhöhle.

Es ist daher die technische Aufgabe der vorliegenden Erfindung, ein Scannen einer Mundhöhle mittels eines Intraoralscanners zu verbessern.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch eine Vakuumfolie zum Scannen einer Mundhöhle gelöst, mit einer Perforationsschicht; einer Faserschicht, die auf der Perforationsschicht angeordnet ist; und einer Texturschicht, die auf der Faserschicht angeordnet ist. Durch die Vakuumfolie wird der technische Vorteil erreicht, dass auch die Form von konturlosem Weichgewebe mittels eines optischen Intraoralscanner erfasst werden kann.

In einer technisch vorteilhaften Ausführungsform der Vakuumfolie weist die Vakuumfolie eine ovale oder eine halbovale Form auf. Dadurch wird beispielsweise der technische Vorteil erreicht, dass die eingesetzte Vakuumfolie die Mundhöhle in einer großen Fläche bedeckt.

In einer weiteren technisch vorteilhaften Ausführungsform der Vakuumfolie weist die Vakuumfolie eine mittige Falte auf. Dadurch wird beispielsweise der technische Vorteil erreicht, dass die Vakuumfolie sich besser in die Mundhöhle einsetzen lässt.

In einer weiteren technisch vorteilhaften Ausführungsform der Vakuumfolie umfasst die Vakuumfolie einen Anschluss zum Anschließen eines Vakuumschlauchs. Dadurch wird beispielsweise der technische Vorteil erreicht, dass auf einfache Weise ein Vakuum erzeugen lässt, durch das die Vakuumfolie an die Mundhöhle gedrückt wird.

In einer weiteren technisch vorteilhaften Ausführungsform der Vakuumfolie weist die Perforationsschicht eine Vielzahl von Öffnungen auf. Dadurch wird beispielsweise der technische Vorteil erreicht, dass sich das Vakuum in der Vakuumfolie effizient ausbreiten kann.

In einer weiteren technisch vorteilhaften Ausführungsform der Vakuumfolie sind die Öffnungen in einem hexagonalen Muster angeordnet. Dadurch wird beispielsweise der technische Vorteil erreicht, dass Luft gleichmäßig abgesaugt werden kann und sich die Vakuumfolie großflächig an die Mundhöhle ansaugt.

In einer weiteren technisch vorteilhaften Ausführungsform der Vakuumfolie weisen die Öffnungen einen Durchmesser zwischen 0.1 mm und 2 mm auf. Dadurch wird beispielsweise ebenfalls der technische Vorteil erreicht, dass die Luft schnell und effizient aus der Vakuumfolie entfernt werden kann.

In einer weiteren technisch vorteilhaften Ausführungsform der Vakuumfolie umfasst die Faserschicht Fasern aus Cellulose oder Watte. Dadurch wird beispielsweise der technische Vorteil erreicht, dass sich die Vakuumfolie gut an die Mundhöhle anschmiegt.

In einer weiteren technisch vorteilhaften Ausführungsform der Vakuumfolie weist die Faserschicht eine Dicke zwischen 0.1 mm und 2.0 mm oder zwischen 0.05 mm und 1.0 mm auf. Dadurch wird beispielsweise der technische Vorteil erreicht, dass der Tragekomfort der Vakuumfolie verbessert wird.

In einer weiteren technisch vorteilhaften Ausführungsform der Vakuumfolie weist die Texturschicht ein Rautenmuster, ein Punktemuster oder ein Kreuzmuster auf. Dadurch wird beispielsweise der technische Vorteil erreicht, dass sich die Form der Mundhöhle besonders gut scannen lässt.

In einer weiteren technisch vorteilhaften Ausführungsform der Vakuumfolie umfasst die Texturschicht eine elastische Kunststofffolie. Dadurch wird beispielsweise der technische Vorteil erreicht, dass sich die Vakuumfolie gut an die Form der Mundhöhle anpasst.

In einer weiteren technisch vorteilhaften Ausführungsform der Vakuumfolie ist die Kunststofffolie aus Polyolefin oder einem thermoplastischen Elastomer gebildet. Dadurch wird beispielsweise der technische Vorteil erreicht, dass besonders geeignete Materialien verwendet werden.

In einer weiteren technisch vorteilhaften Ausführungsform der Vakuumfolie weist die Kunststofffolie eine Dicke zwischen 0.05 mm und 1,0 mm auf. Dadurch wird beispielsweise der technische Vorteil erreicht, dass die Vakuumfolie eine gute Festigkeit aufweist.

Gemäß einem zweiten Aspekt wird die technische Aufgabe durch ein Verfahren zum Scannen einer Mundhöhle gelöst, mit den Schritten eines Einlegens einer Vakuumfolie nach dem ersten Aspekt in die Mundhöhle; eines Absaugens der Luft aus der Vakuumfolie; und eines Scannens der Textur der Texturschicht mittels eines Scanners. Dadurch werden die gleichen technischen Vorteile wie durch die Vakuumfolie nach dem ersten Aspekt erreicht.

In einer technisch vorteilhaften Ausführungsform des Verfahrens erfolgt das Absaugen der Luft über einen Vakuumschlauch. Dadurch wird beispielsweise der technische Vorteil erreicht, dass sich Luft der Vakuumfolie auf einfache Weise entfernen lässt.

Ausführungsbeispiele der Erfindung sind in den Zeichnungen dargestellt und werden im Folgenden näher beschrieben.

Es zeigen:
- Fig. 1: eine perspektivische Ansicht einer Mundhöhle und einer Vakuumfolie;
- Fig. 2: eine perspektivische Ansicht des Aufbaus der Vakuumfolie; und
- Fig. 3: ein Blockdiagramm eines Verfahrens zum Scannen einer Mundhöhle.

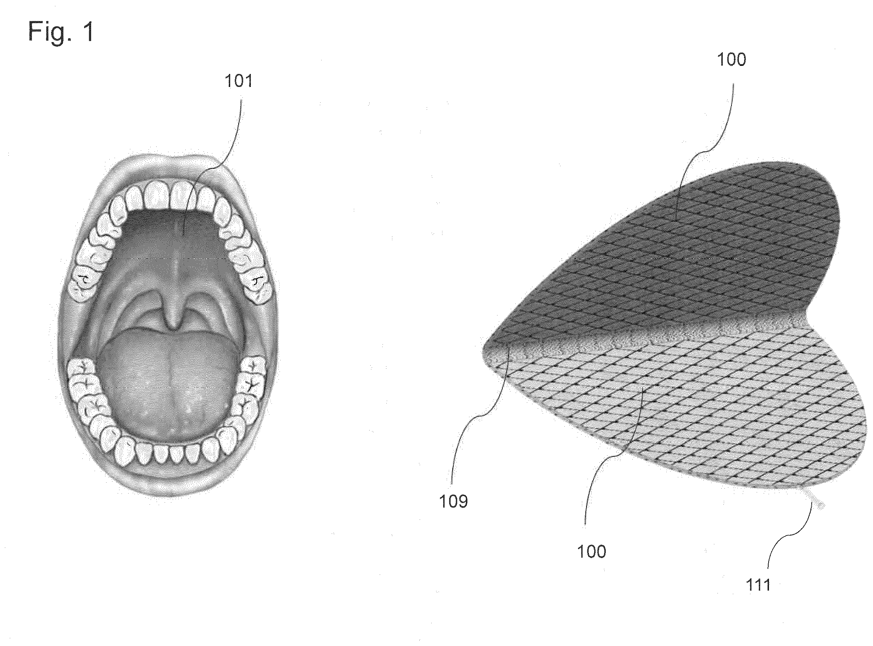

Fig. 1 zeigt eine perspektivische Ansicht einer Mundhöhle 101 und einer Vakuumfolie 100. Die Vakuumfolie 100 wird in den Mundraum eines Patienten gelegt und an der Mundschleimhaut durch Anlegen eines Vakuums festgesaugt. Danach kann die Textur der Vakuumfolie 100 mittels eines Intraoralscanners optisch erfasst werde, um die Form des Mundraums zu bestimmen.

Die Vakuumfolie 100 weist eine ovale Form mit einer mittigen Falte 109 auf. Hierdurch kann die Vakuumfolie 100 auf einfache Weise in den Mund eines Patienten eigeführt werden und passt sich gut an den Mundraum an. Die Vakuumfolie 100 hat beispielsweise eine Länge von min. 30 mm und eine Breite von min. 50 mm. Es kann auch eine halbovale Form der Vakuumfolie verwendet werden,

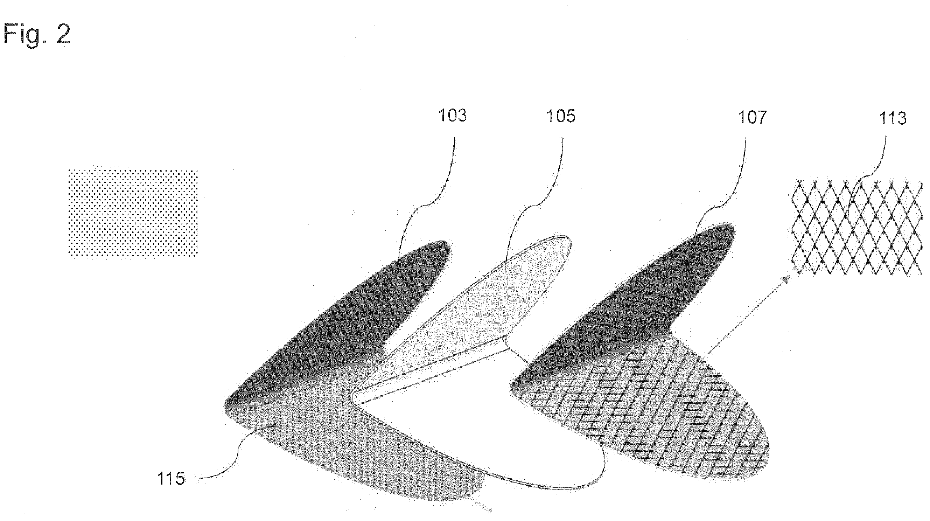

Fig. 2 zeigt eine perspektivische Ansicht des Aufbaus der Vakuumfolie 100. Die Vakuumfolie 100 umfasst mindestens drei unterschiedliche Schichten in einem Sandwichverbund.

Die Perforationsschicht 103 ist perforiert und liegt konturgetreu dem Kiefer an. Diese weist hierzu eine Vielzahl von Öffnungen 115 auf. Die Öffnungen 115 sind in einem hexagonalen Muster angeordnet. Der Abstand der Öffnungen 115 zueinander beträgt beispielsweise 20 mm. Die Größe der Öffnungen beträgt beispielsweise 50 mm Die Perforationsschicht 103 ist beispielsweise aus Polyolefin oder einem thermoplastischen Elastomer gebildet und weist eine Dicke von 0.05 mm bis 2.0 mm auf.

Die Faserschicht 105 ist auf der Perforationsschicht 103 angeordnet ist und umfasst ein saugfähiges und luftdurchlässiges Material, das zusätzlich für eine Trockenlegung geeignet ist. Die Faserschicht 115 ist beispielsweise aus Cellulose oder Watte gebildet und umfasst Fasern aus Watte. Die Dicke der Faserschicht 105 beträgt beispielsweise 0.05 mm bis 2.0 mm.

Die Texturschicht 107 ist wiederum auf der Faserschicht 107 angeordnet und umfasst eine luftdichte Barrierefolie, die mit einem Geometriemuster versehen ist. Die Texturschicht 107 ist beispielsweise aus einem luftundurchlässigen und elastischen Kunststoff gebildet, wie beispielsweise einem thermoplastischen Elastomer oder Polyolefin Die Texturschicht 107 kann beispielsweise eine Dicke von 0.05 mm - 1.0 mm aufweisen. Das aufgebrachte Geometriemuster kann beispielsweise ein Rautenmuster, ein Linienmuster oder ein Farbmuster sein.

Die Perforationsschicht 103, die Faserschicht 105, die Texturschicht 107 sind an deren Außenumfang miteinander verbunden. An der Vorderseite der Vakuumfolie 100 befindet sich ein Anschluss 111, über den die Luft aus der Vakuumfolie 100 abgesaugt werden kann. Hierzu kann ein Schlauch angeschlossen werden, über den die Luft abgesaugt wird.

Wird die Luft aus der Perforationsschicht 103 und der Faserschicht 105 abgesaugt, wird die Vakuumfolie 100 an die Mundhöhle 101 angedrückt. Das Geometriemuster der anliegenden Vakuumfolie 100 kann dann gut von einem Intraoralscanner erfasst werden.

Durch die Verwendung der Vakuumfolie 100 sind weniger Arztbesuche erforderlich und es kann von Anfang bis zum Ende der Behandlung ein digitaler Arbeitsablauf eingesetzt werden. Die Herstellung von physischen Abdrucken entfällt in diesem Fall.

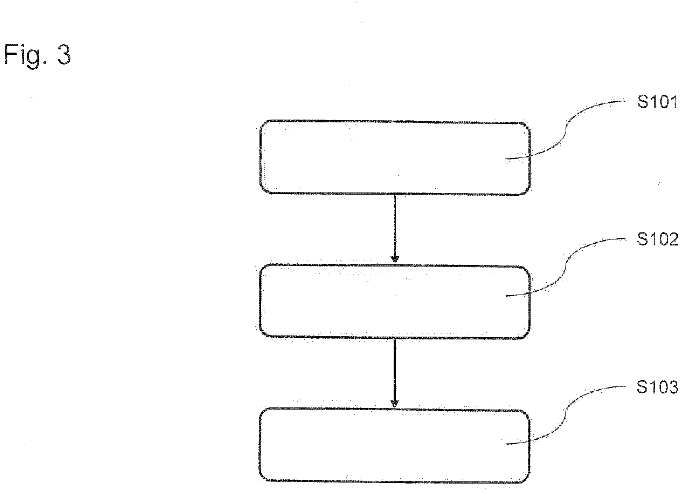

Fig. 3 zeigt ein Blockdiagramm eines Verfahrens zum Scannen einer Mundhöhle. In Schritt S101 wird die Vakuumfolie 100 in die Mundhöhle eingelegt. Danach wird in Schritt S102 die Luft aus der Vakuumfolie 100 abgesaugt. Danach wird in Schritt S103 die Textur 113 der Texturschicht 107 mittels eines Scanners gescannt. Dadurch kann eine genaue Form der Mundhöhle 101 beim Scannen erhalten werden.

Durch das Verfahren unter Verwendung der Vakuumfolie kann ein zahnloser Kiefer mittels eines Intraoralscanners gescannt werden. In diesem Fall muss nicht mehr zuvor ein Andruck hergestellt werden, der anschließend gescannt wird.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Vakuumfolie
- 101: Mundhöhle
- 103: Perforationsschicht
- 105: Faserschicht
- 107: Texturschicht
- 109: Falte
- 111: Anschluss
- 113: Textur
- 115: Öffnung

## Patentansprüche

1. Vakuumfolie (100) zum Scannen einer Mundhöhle (101), mit:
- einer Perforationsschicht (103);
- einer Faserschicht (105), die auf der Perforationsschicht (103) angeordnet ist; und
- einer Texturschicht (107), die auf der Faserschicht (105) angeordnet ist.

2. Vakuumfolie (100) nach Anspruch 1, wobei die Vakuumfolie (100) eine ovale oder eine halbovale Form aufweist.

3. Vakuumfolie (100) nach einem der vorangehenden Ansprüche, wobei die Vakuumfolie (100) eine mittige Falte (109) aufweist.

4. Vakuumfolie (100) nach einem der vorangehenden Ansprüche, wobei die Vakuumfolie (100) einen Anschluss (111) zum Anschließen eines Vakuumschlauchs umfasst.

5. Vakuumfolie (100) nach einem der vorangehenden Ansprüche, wobei die Perforationsschicht (103) eine Vielzahl von Öffnungen (115) aufweist.

6. Vakuumfolie (100) nach Anspruch 5, wobei die Öffnungen (115) in einem hexagonalen Muster angeordnet sind.

7. Vakuumfolie (100) nach Anspruch 5 oder 6, wobei die Öffnungen (115) einen Durchmesser zwischen 0.1 mm und 2 mm aufweisen.

8. Vakuumfolie (100) nach einem der vorangehenden Ansprüche, wobei die Faserschicht (105) Fasern aus Cellulose oder Watte umfasst.

9. Vakuumfolie (100) nach einem der vorangehenden Ansprüche, wobei die Faserschicht (105) eine Dicke zwischen 0.1 mm und 2.0 mm aufweist.

10. Vakuumfolie (100) nach einem der vorangehenden Ansprüche, wobei die Texturschicht (107) ein Rautenmuster, ein Punktemuster oder ein Kreuzmuster aufweist.

11. Vakuumfolie (100) nach einem der vorangehenden Ansprüche, wobei die Texturschicht (107) durch eine elastische Kunststofffolie umfasst.

12. Vakuumfolie (100) nach Anspruch 11, wobei die Kunststofffolie aus Polyolefin oder einem thermoplastischen Elastomer gebildet ist

13. Vakuumfolie (100) nach einem der Ansprüche 11 oder 12, wobei die Kunststofffolie eine Dicke zwischen 0.05 mm und 1.0 mm aufweist.

14. Verfahren zum Scannen einer Mundhöhle, mit den Schritten:
- Einlegen (S101) einer Vakuumfolie (100) nach einem der Ansprüche 1 bis 13 in die Mundhöhle;
- Absaugen (S102) der Luft aus der Vakuumfolie (100); und
- Scannen (S103) der Textur (113) der Texturschicht (107) mittels eines Scanners.

15. Verfahren nach Anspruch 14, wobei das Absaugen der Luft über einen Vakuumschlauch erfolgt.

## Claims

1. A vacuum film (100) for scanning an oral cavity (101), comprising:
- a perforation layer (103);
- a fiber layer (105) arranged on the perforation layer (103); and
- a texture layer (107) arranged on the fiber layer (105).

2. The vacuum film (100) according to claim 1, wherein the vacuum film (100) has an oval or a semi-oval shape.

3. The vacuum film (100) according to any one of the preceding claims, wherein the vacuum film (100) comprises a central fold (109).

4. The vacuum film (100) according to any one of the preceding claims, wherein the vacuum film (100) comprises a connection (111) for connecting a vacuum hose.

5. The vacuum film (100) according to any one of the preceding claims, wherein the perforation layer (103) comprises a plurality of openings (115).

6. The vacuum film (100) according to claim 5, wherein the openings (115) are arranged in a hexagonal pattern.

7. The vacuum film (100) according to claim 5 or 6, wherein the openings (115) have a diameter between 0.1 mm and 2 mm.

8. The vacuum film (100) according to any one of the preceding claims, wherein the fiber layer (105) comprises fibers of cellulose or cotton wool.

9. The vacuum film (100) according to any one of the preceding claims, wherein the fiber layer (105) has a thickness between 0.1 mm and 2.0 mm.

10. The vacuum film (100) according to any one of the preceding claims, wherein the texture layer (107) comprises a diamond pattern, a dot pattern or a cross pattern.

11. The vacuum film (100) according to any one of the preceding claims, wherein the texture layer (107) comprises an elastic plastic film.

12. The vacuum film (100) according to claim 11, wherein the plastic film is formed from polyolefin or a thermoplastic elastomer.

13. The vacuum film (100) according to any one of claims 11 or 12, wherein the plastic film has a thickness between 0.05 mm and 1.0 mm.

14. A method for scanning an oral cavity, comprising the steps of:
- inserting (S101) a vacuum film (100) according to any one of claims 1 to 13 into the oral cavity;
- sucking off (S102) the air from the vacuum film (100); and
- scanning (S103) the texture (113) of the texture layer (107) by means of a scanner.

15. The method according to claim 14, wherein the air is sucked off via a vacuum hose.

## Revendications

1. Film de mise sous vide (100) pour scanner une cavité buccale (101), comprenant :
- une couche de perforation (103) ;
- une couche de fibres (105) disposée sur la couche de perforation (103) ; et
- une couche de texture (107) disposée sur la couche de fibres (105).

2. Film de mise sous vide (100) selon la revendication 1, où le film de mise sous vide (100) présente une forme ovale ou semi-ovale.

3. Film de mise sous vide (100) selon l'une des revendications précédentes, où le film de mise sous vide (100) présente un pli central (109).

4. Film de mise sous vide (100) selon l'une des revendications précédentes, où le film de mise sous vide (100) comprend un raccord (111) pour le raccordement d'un tuyau à vide.

5. Film de mise sous vide (100) selon l'une des revendications précédentes, où la couche de perforation (103) présente une pluralité d'ouvertures (115).

6. Feuille d'aluminium sous vide (100) selon la revendication 5, où les ouvertures (115) sont disposées selon un motif hexagonal.

7. Film de mise sous vide (100) selon la revendication 5 ou 6, où les ouvertures (115) présentent un diamètre compris entre 0,1 mm et 2 mm.

8. Film de mise sous vide (100) selon l'une des revendications précédentes, où la couche de fibres (105) comprend des fibres de cellulose ou de ouate.

9. Film de mise sous vide (100) selon l'une des revendications précédentes, où la couche de fibres (105) présente une épaisseur comprise entre 0,1 mm et 2,0 mm.

10. Film de mise sous vide (100) selon l'une des revendications précédentes, où la couche de texture (107) présente un motif en losange, un motif de points ou un motif en croix.

11. Film de mise sous vide (100) selon l'une des revendications précédentes, où la couche de texture (107) est constituée d'un film plastique élastique.

12. Film de mise sous vide (100) selon la revendication 11, où le film plastique est formé de polyoléfine ou d'un élastomère thermoplastique

13. Film de mise sous vide (100) selon l'une des revendications 11 ou 12, où le film plastique présente une épaisseur comprise entre 0,05 mm et 1,0 mm.

14. Procédé de balayage d'une cavité buccale, comprenant les étapes consistant à :
- insérer (S101) un film de mise sous vide (100) dans la cavité buccale selon l'une des revendications 1 à 13 ;
- aspirer (S102) l'air du film de mise sous vide (100) ; et
- numériser (S103) la texture (113) de la couche de texture (107) à l'aide d'un scanner.

15. Procédé selon la revendication 14, où l'aspiration de l'air se fait à l'aide d'un tuyau à vide.
